# FASCICULE DE BREVET EUROPEEN

(11) **EP 3 052 478 B1**
(45) Date de publication et mention de la délivrance du brevet: **20.06.2018**
(21) Numéro de dépôt: 14784320.5
(22) Date de dépôt: 22.09.2014
(51) Int. Cl.: C07D 233/90, H01M 10/0525, H01M 10/0568

(54) **COMPOSITION COMPRENANT UN SEL D'ANION PENTACYCLIQUE ET SON UTILISATION COMME ÉLECTROLYTE DE BATTERIE**
ZUSAMMENSETZUNG UMFASSEND EIN SALZ MIT PENTAZYKLISCHEM ANION UND DIE VERWENDUNG DERSELBEN ALS ELEKTROLYTEN FÜR EINE BATTERIE
COMPOSITION COMPRISING A PENTACYCLIC ANION SALT AND THE USE THEREOF AS BATTERY ELECTROLYTE

(30) Priorité: 03.10.2013 FR 1359602
(43) Date de publication de la demande: 10.08.2016
(60) Demande divisionnaire: 18172572.2
(73) Titulaire: Arkema France, 92700 Colombes (FR)
(72) Inventeur: SCHMIDT, Grégory, F-69700 Saint Andéol le Château (FR); VAN HEMELRYCK, Bruno, F-69630 Chaponost (FR)
(74) Mandataire: Chahine, Audrey Claire
(86) Numéro de dépôt international: PCT/FR2014/052348
(87) Numéro de publication internationale: WO 2015/049435

(56) Documents cités:
- WO-A1-2010/023413
- WO-A1-2013/072591
- L. NIEDZICKI ET AL: "New type of imidazole based salts designed specifically for lithium ion batteries", ELECTROCHIMICA ACTA, vol. 55, no. 4, 13 mai 2009 (2009-05-13), pages 1450-1454, XP026867410, ISSN: 0013-4686 [extrait le 2009-05-13]
- M. BUKOWSKA ET AL: "Synthesis of 4,5-dicyanoimidazoles", POLISH JOURNAL OF CHEMISTRY , vol. 78, no. 3 1 janvier 2004 (2004-01-01), pages 417-422, XP008104420, ISSN: 0137-5083 Extrait de l'Internet: URL:http://ichf.edu.pl/pjch/pj-2004/pj-200 4-03a.pdf

## Description

### DOMAINE DE L'INVENTION

La présente invention concerne la composition de sel d'anion pentacyclique, et notamment de 2-trifluorométhyl-4,5-dicarbonitrile-imidazolate de lithium, pour électrolytes de batterie.

### ARRIERE-PLAN TECHNIQUE

Une batterie lithium-ion comprend au moins une électrode négative, une électrode positive, un séparateur et un électrolyte. L'électrolyte est constitué d'un sel de lithium dissous dans un solvant qui est généralement un mélange de carbonates organiques, afin d'avoir un bon compromis entre la viscosité et la constante diélectrique.

Parmi les sels les plus utilisés figure l'hexafluorophosphate de lithium (LiPF6), qui possède beaucoup des nombreuses qualités requises mais présente le désavantage de se dégrader sous forme de gaz d'acide fluorhydrique. Cela pose des problèmes de sécurité, notamment dans le contexte de l'utilisation prochaine des batteries lithium-ion pour les véhicules particuliers.

D'autres sels ont donc été développés pour fournir des électrolytes de batteries Li-ion, et notamment le LiTDI (2-trifluorométhyl-4,5-dicarbonitrile-imidazolate de lithium) et le LiPDI (2-pentafluoroéthyl-4,5-dicarbonitrile-imidazolate de lithium), ainsi que cela est enseigné dans le document WO 2010/023413. Ces sels présentent l'avantage de posséder moins d'atomes de fluor et de comporter des liaisons fortes carbone-fluor en lieu et place des liaisons plus faibles phosphore-fluor du LiPF6. En outre, ces sels présentent de très bonnes conductivités de l'ordre de 6 mS/cm, une très bonne dissociation entre l'anion imidazolate et le cation lithium. Le document WO 2013/072591, ainsi que les articles publiés dans Electrochimica Acta, vol. 55, no. 4, 13 mai 2009, pages 1450-1454, et Polish Journal of Chemistry, vol. 78, no. 3, 1er janvier 2004, pages 417-422, décrivent également des procédés de préparation de sels de lithium d'anions pentacycliques, tels que LiTDI ou LiPDI.

Le document WO 2010/023413 propose plusieurs voies de synthèse pour la fabrication de ces anions pentacycliques, dont l'une consiste en la condensation du diaminomaléonitrile (DAMN) sur un dérivé d'acide tel qu'un anhydride d'acide fluoré, suivie d'un échange proton/lithium. Le sel obtenu est alors purifié pour atteindre une composition optimale vis-à-vis de ses performances au sein d'un électrolyte pour batterie Li-ion.

Toutefois, les sels issus après l'étape de purification du WO 2010/023413 posent problème dans l'application des batteries. Les méthodes d'analyses courantes n'ont pas permis d'identifier et/ou de quantifier les impuretés organiques et inorganiques, présentes dans les sels, pouvant nuire à leur utilisation en tant qu'électrolyte dans les batteries.

La présente invention a pour premier objet une composition de sel d'anion pentacyclique ne présentant pas les inconvénients précités.

La présente invention a également pour objet l'utilisation de cette composition comme électrolyte dans les batteries.

La présente invention a en outre pour objet un procédé de fabrication d'obtention de la composition selon le premier objet.

La présente invention fournit le seuil en teneur de composés ioniques et non ioniques présents dans la composition de sels pentacycliques qui convient pour les performances de l'électrolyte en batterie, en particulier la qualité d'établissement de la SEI (Solid Electrolyte Interface) ainsi que la rétention de capacité de la batterie au cours des cycles charge-décharge.

### RESUME DE L'INVENTION

La présente invention concerne une composition comprenant un sel de lithium de composé imidazole de formule (I) : 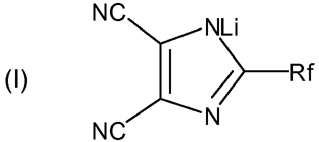 dans laquelle Rf représente un groupement alkyle fluoré ayant de 1 à 5 atome(s) de carbone, ou un atome de fluor :

Selon un mode de composition, Rf représente CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F_{7,} C₄H₄F₅, C₅F₁₁, C₃F₆OCF₃, C₂F₄OCF₃, C₂H₂F₂OCF₃ ou CF₂OCF₃, de préférence CF₃, C₂F₅, C₂F₄OCF₃, C₂H₂F₂OCF₃ ou CF₂OCF₃.

Selon un autre mode de composition, Rf représente F.

La composition, selon la présente invention, comprend un sel de formule (I) et au moins un cation choisi dans le groupe A constitué par le sodium, le potassium, le calcium, le fer, le magnésium, le manganèse, le strontium, le vanadium, l'ammonium, l'argent, l'aluminium, l'arsenic, le barium, le silicium, le cadmium, le cobalt, le chrome, le cuivre, le nickel, le plomb, l'antimoine, le sélénium, l'étain, et le titane et au moins un anion choisi dans le groupe B constitué par le fluorure, le chlorure, le nitrate, le sulfate, le phosphate, l'acétate, le formiate, le trifluoroacétate, le pentafluoroacétate et l'anion de formule (II) 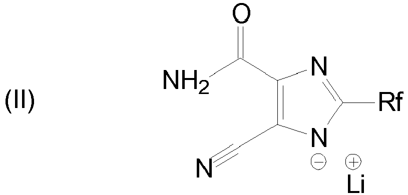 avec la quantité totale des cation(s) et anion(s) étant supérieure à 0 et d'au plus 1 % en poids de la composition.

La présente invention permet de surmonter les inconvénients de l'état de la technique, en particulier en fournissant les performances électrolytiques optimales à la première charge et leur préservation au cours des cycles de charge-décharge. La composition selon la présente invention assure une bonne SEI (Solid Electrolyte Interface) à la première charge ainsi qu'une excellente rétention de capacité au fil des cycles de travail de la batterie.

### DESCRIPTION DETAILLEE DE L'INVENTION

La composition selon la présente invention comprend (i) un sel de lithium de composé imidazole de formule (I) : 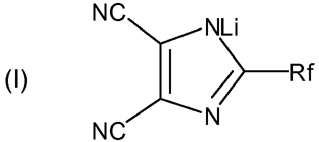 dans laquelle Rf représente un groupement alkyle fluoré ayant de 1 à 5 atome(s) de carbone, ou un atome de fluor et (ii) au moins un cation choisi dans le groupe A constitué par le sodium, le potassium, le calcium, le fer, le magnésium, le manganèse, le strontium, le vanadium, l'ammonium, l'argent, l'aluminium, l'arsenic, le barium, le silicium, le cadmium, le cobalt, le chrome, le cuivre, le nickel, le plomb, l'antimoine, le sélénium, l'étain, et le titane et (iii) au moins un anion choisi dans le groupe B constitué par le fluorure, le chlorure, le nitrate, le sulfate, le phosphate, l'acétate, le formiate, le trifluoroacétate, le pentafluoroacétate et l'anion de formule (II), avec la quantité totale des cation(s) et anion(s) étant supérieure à 0 et d' au plus 1 % en poids de la composition.

Selon les modes de réalisation :
- la quantité de sodium est comprise entre 0 et 500 ppm et de préférence compris entre 0 et 100 ppm;
- la quantité de potassium est comprise entre 0 et 1000 ppm et de préférence comprise entre 0 et 500 ppm et avantageusement comprise entre 0 et 100 ppm;
- la quantité de calcium est comprise entre 0 et 70 ppm;
- la quantité de fer est comprise entre 0 et 10 ppm;
- la quantité de magnésium est comprise entre 0 et 10 ppm;
- la quantité de manganèse est comprise entre 0 et 5 ppm;
- la quantité de strontium est comprise entre 0 et 5 ppm;
- la quantité de vanadium est comprise entre 0 et 10 ppm;
- la quantité totale des cations suivants : Ag, Al, As, Ba, Si, Cd, Co, Cr, Cu, Ni, Pb, Sb, Se, Sn, Sr, Ti, Zn est comprise entre 0 et 200 ppm ;
- la quantité d'ammonium (NH₄⁺) est comprise entre 0 et 10 ppm;
- la quantité de fluorure est comprise entre 0 et 100 ppm et de préférence comprise entre 0 et 10 ppm;
- la quantité d'acétate est comprise entre 0 et 30 ppm et de préférence comprise entre 0 et 5 ppm;
- la quantité de formiate est comprise entre 0 et 200 ppm et de préférence comprise entre 0 et 10 ppm;
- la quantité de chlorure est comprise entre 0 et 500 ppm et de préférence comprise entre 0 et 100 ppm et avantageusement comprise entre 0 et 50 ppm;
- la quantité de nitrate est comprise entre 0 et 150 ppm et de préférence entre 0 et 100 ppm et avantageusement entre 0 et 50 ppm;
- la quantité de sulfate est comprise entre 0 et 500 ppm et de préférence comprise entre 0 et 150 ppm, et avantageusement comprise entre 0 et 25 ppm;
- la quantité de phosphate est comprise entre 0 et 100 ppm et de préférence entre 0 et 10 ppm;
- la quantité de trifluoroacétate est comprise entre 0 et 100 ppm ;
- la quantité de pentafluoroacétate est comprise entre 0 et 200 ppm et de préférence entre 0 et 100 ppm ;
- la quantité de l'anion de (II) est comprise entre 0 et 600 ppm et de préférence entre 0 et 400 ppm;
- Rf représente F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F_{7,} C₄H₄F₅, C₅F₁₁, C₃F₆OCF₃, C₂F₄OCF₃, C₂H₂F₂OCF₃ ou CF₂OCF₃, de préférence CF₃, C₂F₅, C₂F₄OCF₃, C₂H₂F₂OCF₃ ou CF₂OCF₃.

La demanderesse a découvert que les compositions préférées selon l'invention peuvent être caractérisées par une ligne de base du spectre Raman qui dévie très peu par rapport à l'horizontale. L'analyse Raman a été effectuée à l'aide d'un microscope JOBIN YVON dans les conditions suivantes :
Excitation Lazer vert à 532 nm
Diaphragme de confocalisation = 1000 µ
Réseau 600 lignes
Détecteur 2800 cm⁻¹
et les spectres résultent de l'addition de 5 mesures de 16 scans de 1 seconde chacun.

Dans ces conditions, la déviation de la ligne de base du spectre prise entre 4500 cm⁻¹ et 3000 cm⁻¹ et dépourvue de toute raie Raman ne doit pas excéder un certain facteur de pente a par rapport à l'horizontale. Ce facteur est défini par régression linéaire de la ligne de base du spectre entre 4500 cm⁻¹ et 3000 cm⁻¹ avec comme équation *y* = *ax* + *b, y* étant l'intensité, *x* étant la longueur d'onde et a représente la pente de cette droite de régression linéaire par rapport à l'horizontale, *b* représente un décalage en intensité *y* à la longueur d'onde 4500 cm⁻¹ dû à l'effet de la fluorescence causée par les traces d'impuretés. *y* et *b* sont exprimées en unités d'intensité Raman du constructeur. Lorsque le spectre Raman ne présente pas de déviation de ligne de base due à la présence d'impuretés fluorescentes, a = 0. La valeur de *b* dépend de l'instrumentation, en particulier de l'état de vieillissement du laser et une même poudre examinée sur un autre microscope Raman fournirait une valeur de *b*, et par conséquent une intensité Raman y différente.
Par contre la pente a de la droite de régression de la ligne de base du spectre Raman entre 4500 cm⁻¹ et 3000 cm⁻¹ restera sensiblement la même.
La valeur absolue de la pente a entre 4500 cm⁻¹ et 3000 cm⁻¹ est donc de préférence *a* ≤ 25, facteur de pente maximal admissible, de manière encore préférée *a* ≤15 et de manière encore plus préférée *a* ≤5.
Selon un mode de réalisation préféré de l'invention, la composition comprend un sel de formule (I) et au moins un cation, choisi dans le groupe A, et au moins un anion, choisi dans le groupe B, avec la totalité des cation(s) et anion(s) représentant supérieure à 0 et au plus 1 % en poids de la composition et en ce que le sodium et/ou le potassium (est) sont présent(s) en quantité supérieure à 0 ppm et inférieure ou égale à 100 ppm.

Selon un autre mode préféré de l'invention, la composition comprend un sel de formule (I) et au moins un cation, choisi dans le groupe A, et au moins un anion, choisi dans le groupe B, avec la totalité des cation(s) et anion(s) représentant supérieure à 0 et au plus 1 % en poids de la composition et en ce que le trifluoroacétate et /ou le pentafluoroacétate (est) sont présent(s) en quantité supérieure à 0 ppm et inférieure ou égale à 100 ppm.

Selon un mode de réalisation particulièrement préféré de l'invention, la composition comprend un sel de formule (I) et au moins un cation, choisi dans le groupe A, et au moins un anion, choisi dans le groupe B, avec la totalité des cation(s) et anion(s) représentant supérieure à 0 et au plus 1 % en poids de la composition et en ce que le sodium et/ou le potassium (est) sont présent(s) en quantité supérieure à 0 ppm et inférieure ou égale à 100 ppm et en ce que le trifluoroacétate et /ou le pentafluoroacétate (est) sont présent(s) en quantité supérieure à 0 ppm et inférieure ou égale à 100 ppm. Quel que soit le mode de réalisation de l'invention, le sel de formule (I) représente au moins 99 %, de préférence au moins 99,9 % et avantageusement au moins 99,95 % en poids de la composition.

De préférence, la composition comprenant le sel de formule (I) lorsqu'elle est présente à une concentration d'un mole par litre d'un solvant, non absorbant dans le visible, présente une coloration Hazen < 10.

Comme solvant non absorbant dans le visible, on peut citer notamment l'acétonitrile, l'éthanol, l'acétate d'éthyle, l'eau.

De préférence, le composé de formule (I) est le 2-trifluorométhyl-4,5-dicarbonitrile-imidazolate de lithium.

Lorsque le composé de formule (I) est le 2-trifluorométhyl-4,5-dicarbonitrile-imidazolate de lithium, le lithium représente entre 3,4 et 3,8 % en poids, de préférence entre 3,55 et 3,65 % en poids et avantageusement entre 3,59 et 3,61 % en poids de la composition selon l'invention.

### Préparation de l'imidazolate de lithium

La présente invention a également pour objet un procédé d'obtention de la composition selon le premier objet. Ce procédé comprend au moins une étape de traitement d'une solution de sel renfermant le composé de formule (I), préalablement préparée, sur du charbon actif suivie d'au moins une étape de recristillation de la solution traitée.

La solution de sel renfermant le composé de formule (I) peut être préparée selon la méthode décrite dans le document WO 2010/023413 (page 10 ligne 28 à page 12 ligne 16), à savoir préparation d'un acide dans une première étape à partir du diaminomaléonitrile par condensation avec anhydride ou aldéhyde O=CHR_{f} ou cétal suivie d'une deuxième étape au cours de laquelle l'acide est transformé en sel de lithium. Cette solution peut également être préparée selon la méthode décrite dans le document WO 2013/072591.

Le procédé de préparation d'une composition de sel de formule (I) tel que décrite ci-dessus comprend (i) au moins une étape de traitement d'une solution résultant de la réaction d'un composé imidazole de formule (III), en présence d'un solvant (S), avec une base lithiée, de préférence choisie parmi l'hydrure de lithium, le carbonate de lithium, l'hydroxyde de lithium et les combinaisons de ceux-ci, sur du charbon actif ; (ii) au moins une étape de séparation du charbon actif pour donner une solution de composition de sel de formule (I) dans le solvant (S) et (iii) au moins une étape de concentration de la solution obtenue à l'étape (ii) suivie d'au moins une étape de refroidissement jusqu'à la formation de cristaux et (iv) au moins une étape d'essorage et/ou de filtration suivie d'au moins une étape de séchage.

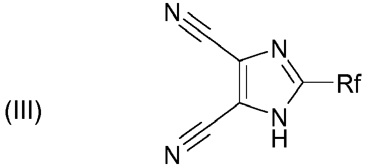

De préférence l'étape de refroidissement est réalisée lorsque la solution est concentrée d'un facteur volumique compris entre 50 et 2, de préférence entre 20 et 2 et de façon encore préférée entre 15 et 2.

Selon une variante du procédé, la solution récupérée à l'étape (ii) est évaporée à sec, puis le solide obtenu est mis en solution dans un solvant (S), différent de celui utilisé pour l'étape (i), avant l'étape de cristallisation (iii) décrite ci-dessus.

La température de refroidissement peut être comprise entre -150 et 0°C, de préférence entre -12 et -2°C.

Le rendement typique de recristallisation pour le LiTDi dans ces conditions est de l'ordre de 90 %.

Par exemple, le LiTDI est obtenu lorsque Rf représente un groupement trifluorométhyle, et le LiPDI lorsque Rf représente un groupement pentafluoroéthyle.

Comme solvant (S), on peut citer notamment l'eau, ou un solvant organique, de préférence choisi parmi l'acétonitrile, les alcools tels que le méthanol, l'éthanol, l'isopropanol, le n-butanol, l'alcool benzylique, l'acétate d'éthyle, les carbonates d'alkyle, tels que l'éthylène ou le diméthyle carbonates, et le tetrahydrofurane (THF).

De préférence, on utilise un solvant organique pour l'étape réactionnelle de lithiation d'un composé_imidazole de formule (III) et/ou l'étape de traitement au charbon actif. Le solvant pour la réaction de lithiation peut être différent de celui de l'étape de traitement par le charbon actif.

Avantageusement, on opère en présence de l'acétonitrile pour l'étape (i) de traitement au charbon actif et/ou pour la remise en solution après l'étape d'évaporation à sec.

Le charbon actif est de préférence choisi parmi les grades de charbons commerciaux dits physiques, c'est-à-dire activés uniquement à la vapeur d'eau à haute température. Parmi les charbons physiques, ceux issus de matière première végétale dite « tendre » telle que le pin, sont particulièrement préférés.

Le charbon actif peut être en poudre ou en grain, ou sous forme de média filtrants composites obtenus par mélangeage du charbon en poudre avec un liant inerte aux solvants organiques précités ou à l'eau. Le charbon actif sous forme de grains ou de filtres est toutefois préféré pour limiter les poussières lors de la mise en oeuvre.

La porosité du charbon actif, mesurée par N₂-BET, est de préférence comprise entre 900 et 1800 m²/g, avantageusement comprise entre 900 et 1300 m²/g et plus particulièrement comprise entre 900 et 1100 m²/g.

La porosité principale du charbon actif peut être de nature microporeuse, mésoporeuse, ou macroporeuse. La mésoporosité est préférée.

La granulométrie D10 du charbon actif utilisé pour le traitement est de préférence comprise entre 5 et 10 µm, avantageusement comprise entre 5 et 8 µm.

La granulométrie D50 du charbon actif utilisé pour le traitement est de préférence comprise entre 20 et 40 µm, avantageusement comprise entre 25 et 35 µm. Une granulométrie D50 comprise entre 28 et 30 µm est particulièrement préférée.

La teneur en cendres est de préférence inférieure ou égale à 5% en poids, avantageusement inférieure ou égale à 3% en poids pour les charbons activés à la vapeur d'eau.

Pour les charbons physiques lavés avec de l'acide, le taux de cendre est de préférence inférieur à 1% en poids.

Le pH initial du charbon, mesuré sur le filtrat obtenu après mise en solution de 4g dans 100ml d'eau décarbonatée par ébullition, suivie de l'ébullition pendant 10 mn et puis filtration à chaud, est de préférence compris entre 10 et 6 et avantageusement compris entre 10 et 7.

De préférence, la solution de la composition de sel de formule (I) utilisée pour l'étape de traitement au charbon actif est comprise entre 50 et 500 g de sel pour un litre de solvant, avantageusement comprise entre 100 et 250 g/l. Une solution comprise entre 130 et 200 g de LiTDI pour un litre d'acétonitrile a donné de résultats particulièrement intéressants.

De préférence, le ratio massique du charbon mis en jeu/masse de (I) dans la solution à traiter est compris entre 1 : 5 et 1 : 8. Les solutions dans l'acétonitrile, solvant préféré selon l'invention, sont établies avec un ratio massique charbon versus (I) compris entre 1 : 5 et 1 : 8, et de manière préférée entre 1 : 6 et 1 : 8. Ainsi, le traitement par 1g de charbon pour 7,5 g de LiTDI sel solide a donné des résultats intéressants.

La température de traitement par le charbon actif est de préférence comprise entre 0 et 60°C.

Les charbons actifs en poudre de la gamme CECA 2SW et 3SW ou tout média filtrant basé sur ces grades conviennent particulièrement.

Selon un mode de réalisation, le charbon actif est soumis à une lixiviation aqueuse avant l'utilisation à l'étape (i) La lixiviation aqueuse peut être mise en oeuvre avec de l'eau distillée ou permutée et du charbon actif en poudre à l'aide d'un filtre en utilisant une pression allant jusqu'à 3 bar.

A titre d'exemple, la demanderesse a obtenue, pour une solution molaire de sel(I) dans un solvant organique, une coloration Hazen < 10 après plusieurs étapes de traitement par le charbon actif d'une solution initiale colorée brune, typiquement d'absorption 5 à 7 selon la norme Gardner.

A l'issue du traitement par le charbon actif, le sel de formule (I) est soumis à une étape de recristallisation dans un solvant organique.

### Préparation d'un électrolyte

Les composés de formule (I) ainsi préparés, notamment le LiTDI et le LiPDI, peuvent être utilisés pour la préparation d'un électrolyte, en les solubilisant dans un solvant approprié.

Les composés de formule (I) sont par exemple dissous dans un solvant ou un mélange de solvants de 1 à 5 constituant(s) choisi(s) parmi les carbonates suivants : l'éthylene carbonate, le dimethylcarbonate, l'éthylméthylcarbonate, le diéthylcarbonate, le propylene carbonate et les glymes suivants : l'éthylène glycol diméthyléther, le diéthylène glycol diméthyléther, le dipropylène glycol diméthyléther, le diéthylène glycol diéthyléther, le triéthylène glycol diméthyléther, le diéthylène glycol dibutyléther, le tétraéthylène glycol diméthyléther et le diéthylène glycol t-buthylméthyléther. Lorsqu'un mélange de solvant est utilisé, les proportions en masse de chacun des constituants sont de préférence comprises entre 1 et 10 par rapport au constituant en plus faible quantité, plus préférentiellement entre 1 et 8.

La concentration en composé de formule (I) dans l'électrolyte est de préférence de 0,1 mol/l à 5 mol/l et avantageusement de 0,2 mol/l à 2,5 mol/l. Cet électrolyte peut ensuite être utilisé pour la fabrication de batteries ou de cellules de batterie, en le disposant entre une cathode et une anode, de manière connue en soi.

### PARTIE EXPERIMENTALE

La coloration **Hazen** est mesurée selon la norme Hazen, pour une mise en solution de (I) dans un solvant non absorbant dans le domaine du Visible, à une concentration de 1 mole/L, sur un spectrophotomètre Hach Lico150, en cellule de 11 mm de diamètre. Lorsqu'une solution fournit une valeur inférieure à 10 Hazen, le trajet optique est augmenté par l'emploi d'une cuve de 50 mm pour plus de précision. Les valeurs Hazen portées dans les exemples sont les moyennes de 3 mesures d'une même solution.

Les données **ICP-AES** sont obtenues sur un spectromètre ICAP 6500 (Thermo Electronics).

Echantillonnage pour la quantification de Li, K, Na et traces d'éléments de la liste fournie :
L'échantillon de (I) est dissout dans de l'eau ultra-pure. 2 dilutions ont été utilisées : 1g/l pour la détermination du Na, K, et éléments Ag, Al, As, Ba, Si, Cd, Co, Cr, Cu, Ni, Pb, Sb, Se, Sn, Sr, Ti, Zn à l'état de trace, et 0,1g/l pour l'analyse du Lithium.

Analyse qualitative panoramique :
Les conditions ICP-AES appliqués pour l'analyse semi-quantitative dite panoramique des éléments à l'état de trace sont :
Puissance de sortie de la source Plasma : 1150 W
Débit du gaz de débulisation : 0,7 L/min
Débit de refroidissement = 16 L/min
Hauteur de torche : 12 mm
Vitesse de pompe : 50 trm
Bande passante spectrale : 7 pm à 200 nm, 3.5 nm par pixel
Plage de longueurs d'onde : 167 nm à 847 nm.
La méthode ICP-AES de quantification pour mesurer Li, K, Na a utilisé 5 points de calibration.
Pour l'analyse des éléments à l'état de trace Ag, Al, As, Ba, Si, Cd, Co, Cr, Cu, Ni, Pb, Sb, Se, Sn, Sr, Ti, Zn, la méthode semi-quantitative repose sur deux points de calibration.

Pour les deux méthodes, l'étalonnage est réalisé par addition d'étalons dans l'échantillon lui-même de manière à minimiser les effets de matrice.

L'ICP-AES est préférée à la chromatographie cationique en solution aqueuse pour la mesure des éléments Li, Na, K .
Pour les exemples, les conditions d'analyse des anions en **chromatographie ionique (CI)** sont les suivantes :
Appareil Dionex ICS 5000 DUAL
Colonne ASII-HC
Débit 1 ml/min
Eluant KOH en gradient de 2mmole/l à 20 mmole/l en 15 minutes
Détection conductimétrique
Suppresseur ASRS 4 mm avec 50 mA de courant imposé.
Injection de 50 µl de solutions de LiTDI à 5g/l et 10g/l selon la sensibilité requise par espèce anionique présente.
Etalonnage de chaque espèce anionique avec cinq solutions synthétiques allant de 0,05 mg/l jusqu'à 1 mg/l.

Pour les exemples, les conditions d'**analyse RMN** des espèces fluorées telles que (I), (II) en RMN F19, H1, C13 sont les suivantes :

### Equipement :

Les spectres et quantifications RMN ont été effectués sur un spectromètre Bruker AV 400, à 100,62 MHz pour C13 et 376,47 MHz pour F19, sur une sonde de 5 mm de type BBFO⁺.

### Echantillonnage :

Les échantillons de (I) sont dissous dans DMSO-d₆ (environ 30 mg dans 0,6 ml). Dans le cas de la détection de fluorures ou d'ajout de LiF servant à vérifier la présence indésirable de fluorures, le solvant de (I) est D₂O en raison de l'insolubilité de LiF dans le DMSO.

### Quantification :

La quantification relative en RMN F19 est faite par intégration des signaux des espèces fluorées, pondérés du nombre de fluor contribuant au signal, méthode bien connue de l'homme de l'art.

La quantification absolue en RMN F19 est faite par ajout dosé d'α,α,α-trifluorotoluène (TFT), Aldrich dans le tube contenant (I), et par intégration des signaux des espèces fluorées à doser en comparaison de celle des CF₃ de cet étalon interne, selon méthode bien connue de l'homme de l'art. La limite de quantification d'une espèce telle que (II) à la fréquence de 376,47 MHz et la sonde choisie est de l'ordre d'une cinquantaine de ppm.

La **spectrométrie de masse** permet de confirmer la formule brute des impuretés organiques de (I) telles que (II), dans les conditions expérimentales suivantes :

### Equipement :

Les spectres de spectrométrie de masse sont enregistrés sur un spectromètre WATERS QTOF II, utilisant l'ionisation positive et négative des molécules de (I) ou des impuretés organiques telles que (II)

### Echantillonnage :

Les échantillons de (I) avec Rf = CF₃ (LiTDI) sont dissous dans le méthanol à la concentration de 100mg/L.. Ils sont directement injectés dans le spectromètre de masse en utilisant le mode flow injection dans un mélange methanol/eau 70/30 en volume.

Conditions instrumentales :

| **Electrospary positive mode** | **Electrospray negative mode** |
|---|---|
| **Positive electrospray ion source** | **negative etectrospray ion source** |
| Capillary: 3kV | Capillary: 3KV |
| Cone: 30V | Cone: 30V |
| Extractor : 0V | Extractor : 2V |
| Source temperature : 80°C | Source temperature : 80°C |
| dessolvation temperature : 150°C | dessolvation tempe rature : 150°C |
| | |

| **Quadrupole analyser** | |
|---|---|
| Collision energy: 10eV | Collision energy: 10eV |
| Steering: 1.00V | Steering: 1.00V |
| Enfance: 65eV | Entrance: 65eV |
| Pre-filter: 6V | Pre-filter: 6V |
| | |

| **TOF analyser** | |
|---|---|
| Transport: 3.0V | Transport 5.0V |
| Accelerator voltage: 200V | Accelerator voltage: 200V |
| Focus: 0V | Focus: 0.3V |
| Tube lens: 90V | Tube lens: 100V |
| Pusher: 980V | Pusher: 980V |
| TOF : 9.1kV | TOF : 9.1 kV |
| Reflectron: 36.00 | Reflectron: 36.00 |
| Pusher cycle time (µs): auto | Pusher cycle time (µs): 42µs |
| Multiplier : 550 | Multiplier: 550 |
| MCP: 2100V | MCP: 2100V |

### Exemple 1

Dans un réacteur agité double enveloppe de 4 litres muni d'un réfrigérant, d'une ampoule de coulée et d'un thermocouple, on introduit 867,35 g de DAMN de pureté 98% dans 2979,29g de dioxane 1,4 à température ambiante. La suspension est mise sous agitation tandis qu'on introduit au goutte à goutte 1751,57g d'anhydride trifluoroacétique de pureté 99% au moyen de l'ampoule de coulée, tout en maintenant le milieu réactionnel à une température inférieure à 30°C. La suspension se transforme rapidement en une solution colorée brune. Lorsque la totalité de l'anhydride trifluoroacétique est introduite, on augmente la température du milieu réactionnel jusqu'au reflux du dioxane (105°C). Au bout de 30 minutes de reflux, on diminue la température jusqu' à 50°C. On évapore ensuite le contenu du réacteur à l'aide d'un évaporateur rotatif sous vide poussé (<10 mm Hg) jusqu'à l'obtention d'une huile brune. Cette huile est alors reprise à l'eau dans un bécher de 5 litres, avec un ratio massique eau : huile de 1 :1. L'ensemble est mis sous agitation et chauffé à 60°C jusqu'à l'obtention d'une pâte brune homogène, puis refroidi rapidement dans un bain d'eau froide et de glace. Les cristaux obtenus sont alors filtrés et rincés avec 500 ml de toluène puis essorés sous vide.

Les cristaux ainsi essorés sont remis en solution dans l'eau avec un rapport massique cristaux/eau de 1 :1, à une température de 60°C puis refroidi rapidement. Les cristaux obtenus sont filtrés, rincés et essorés comme précédemment.
Après une nouvelle opération de recristallisation, les cristaux sont analysés en RMN F19 et montrent une composition relative en espèces fluorées de 99,7% de HTDI, 0,2% d'amide du HTDI de structure identifiée par RMN et Spectrométrie de masse comme étant le composé de formule (III) et 900 ppm d'acide trifluoroacétique (TFA).

On dissout, à une température d'environ 40°C, les cristaux obtenus précédemment dans l'acétonitrile à raison de 1 g de cristaux pour 3,5 ml de solvant. Puis on y ajoute petit à petit du Li₂CO₃ sous forme solide jusqu'à 290,2 g, et on laisse sous agitation à température ambiante pendant une nuit. Ensuite, on y ajoute de l'acétonitrile pour obtenir une concentration de 1 g de sel pour environ 7,2 ml de solvant.

La solution résultante est ensuite traitée par du charbon actif en poudre 3SW de CECA, préalablement lavé à l'eau permutée, à raison de 1 g de charbon pour 7,5 g de sel LiTDI, pendant 3 heures à température ambiante. A l'issue du traitement, la solution est filtrée.

On effectue de nouveau le traitement avec une nouvelle charge de charbon actif.
Au bout de 4 traitements, on obtient une solution ayant une coloration de 6 Hazen.
Après évaporation de l'acétonitrile et séchage à 120°C sous vide pendant 1 semaine on obtient 1118 g de solide.

L'analyse RMN F19 avec étalonnage interne au α,α,α-trifluorotoluène donne une composition comprenant 99,74% en poids du LiTDI, 0,19% d'amide du LiTDI et 730 ppm de trifluoroacétate de Lithium (TFA-Li).

On prélève 230 g du solide pour le faire dissoudre dans 900 ml d'acétonitrile à 65°C dans un ballon de 2 litres muni d'un réfrigérant et d'un barreau aimanté. Après dissolution totale du solide, on place le ballon dans un bain de glace salée amenant la solution de LiTDi à -2°C, après avoir cessé d'agiter. Le bain de glace est laissé à réchauffer naturellement, le temps de résidence en bain froid étant d'au moins 5 heures, puis le ballon contenant du LiTDI cristallisé et de l'acétonitrile ayant une coloration très légèrement jaunâtre est transféré pour évaporation sous vide modéré dans un bain chauffant à 40°C de façon à concentrer d'un facteur 3 le volume d'acétonitrile.

L'ensemble est ensuite filtré à température ambiante et les cristaux blancs de LiTDI représentent une masse de 210g, soit un rendement de recristallisation acétonitrile de 91,3%. Cette fraction ainsi recristallisée possède une pureté déterminée par RMN F19 et étalonnage interne au α,α,α-trifluorotoluène >99,95% poids en LiTDI et présente de l'ordre de 200 ppm d'amide-Li et moins de 100 ppm de TFA-Li.

L'analyse ICP du solide obtenu, la coloration et les performances en batterie sont résumés dans le tableau ci-après.

| Elément ou impureté (méthode de détermination | Abondance en ppm | Valeur Hazen | SEI : capacité irréversible | Rétention de capacité |
|---|---|---|---|---|
| Li (ICP-AES) | 3,5% | | | |
| Na (ICP-AES) | 30 | | | |
| K (ICP-AES) | ND | | | |
| Ca (ICP-AES) | <10 | | | |
| Fe (ICP-AES) | <5 | | | |
| Sr (ICP-AES) | <5 | | | |
| V (ICP-AES) | <5 | | | 20% après |
| NH4+ (RMN H1) | ND | 6 | 14% (EC/EMC | 720 cycles réalisés à un |
| F- (Cl) | 8 | | 3/7 v/v) | régime de 1C |
| CH3COO- (Cl) | 30 | | | |
| HCOO-(Cl) | 9 | | | |
| Cl- (Cl) | 3 | | | |
| TFA- (Cl) | 71 | | | |
| NO3-(Cl) | 2 | | | |
| SO4 ²⁻ (Cl) | 31 | | | |

Composition du solide en % poids relatifs selon analyse RMN F19 :

| Espèce fluorée | Quantité relative des espèces fluorées |
|---|---|
| (I) | 99,97% |
| (III) | 222 ppm |
| TFA- | 98 ppm |

Composition du solide en % poids absolus selon analyse RMN F19, par étalonnage interne au α,α,α-trifluorotoluene (TFT) d'Aldrich :

| Espèce fluorée | Quantité absolue des espèces fluorées |
|---|---|
| (I) | > 99,95 % |
| (III) | 210 ± 30 ppm * |
| TFA- | 93 ± 10 ppm * |

| | |
|---|---|
| *moyenne de 2 calibrations | |

Résultat RAMAN : la pente en valeur absolue de *y* = *ax*+ *b* est de *a* = 3,93

### Exemple 2

On opère comme précédemment mais à l'échelle pilote pour une quantité de LiTDI visée de 35 kg. On utilise un réacteur de 100 litres à la place du réacteur agité de 4 litres et un réacteur de 400 litres pour les traitements au charbon actif en conditions diluées. Les conditionnements par lavage à l'eau des charbons actifs ainsi que leurs filtrations ont été réalisés sur un filtre Tournaire muni d'une toile en PP de 11µm.

L'analyse ICP du solide obtenu, la coloration et les performances en batterie sont résumés dans le tableau ci-après.

| Elément ou impureté | Abondance en ppm | Valeur Hazen | SEI : capacité irréversible | Rétention de capacité |
|---|---|---|---|---|
| Li (ICP-AES) | 3,6% | | | |
| Na (ICP-AES) | 20 ppm | | | |
| K (ICP-AES) | < LD de 10 ppm, | | | |
| Ca | < LD de 9,4 ppm | | | |
| Fe | < LD 0,5 ppm | | | |
| Sr | < LD de 0,01 ppm | | 16% | 21% au |
| V | < LD de 1,2 ppm | 7 | (EC/EMC 1/1 v/v) | bout de 820 cycles en |
| NH4+ | ND (RMN H1) | | | régime 1C |
| F- (Cl) | ND | | | |
| CH3COO-(Cl) | ND | | | |
| HCOO-(Cl) | ND | | | |
| Cl- (Cl) | ND | | | |
| TFA- | 40 ppm | | | |
| NO3- | 1 ppm | | | |
| SO4 ²⁻ | 12 ppm | | | |
| PO4 ³⁻ | 7 ppm | | | |

Ensemble des mesures ICP-AES des cations

| | **résultat (ppm)** | **LD** |
|---|---|---|
| **LD = limite de détection, en ppmAg** | < LD | 0,9 |
| **Al** | < LD | 0,6 |
| **As** | < LD | 2,2 |
| **B** | < LD | 0,3 |
| **Ba** | < LD | 0,1 |
| **Ca** | < LD | 9,4 |
| **Cd** | < LD | 0,6 |
| **Co** | < LD | 0,8 |
| **Cr** | < LD | 1,0 |
| **Cu** | < LD | 0,7 |
| **Fe** | < LD | 0,5 |
| **Mg** | < LD | 0,4 |
| **Mn** | < LD | 0,1 |
| **Mo** | < LD | 0,5 |
| **P** | 2.40 | 2,2 |
| **Sb** | < LD | 2,2 |
| **Si** | < LD | 1,9 |
| **Sn** | < LD | 1,8 |
| **Sr** | < LD | 0,01 |
| **Ti** | < LD | 0,2 |
| **V** | < LD | 1,2 |
| **Zn** | < LD | 0,2 |

Composition de solide en % poids relatifs selon analyse RMN F19 :

| Espèce fluorée | Quantité relative des espèces fluorées |
|---|---|
| (I) | 99,96% |
| (III) | 315 ppm |
| TFA- | 42 ppm |

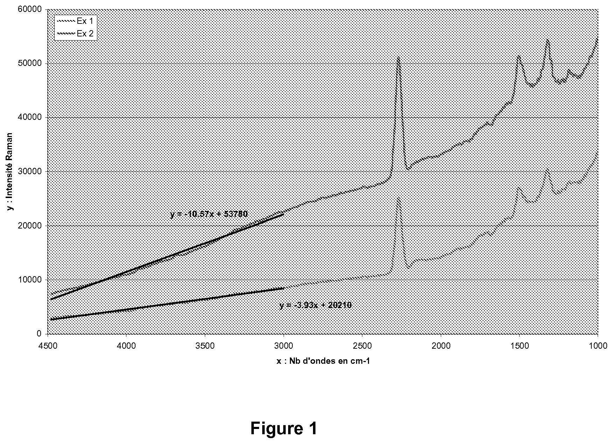

Résultat RAMAN : la pente en valeur absolue de la droite de régression linéaire *y* = *ax* + *b* de la ligne de base du spectre entre x = 4500 cm⁻¹ et x = 3000 cm⁻¹ est de *a* = 10,57
La figure 1 donne les spectres Raman 532 nm des exemples 1 et 2. La régression linéaire de la ligne de base allant de 4500 cm⁻¹ à 3000 cm⁻¹ fournit une droite d'équation y=ax+b, avec y l'intensité Raman, a la pente de la droite par rapport à l'horizontale, x le nombre d'onde Raman, b le décalage de la droite au nombre d'onde x =0 par rapport à l'intensité y=0.

## Revendications

1. Composition comprenant (i) un sel de lithium de composé imidazole de formule (I) : 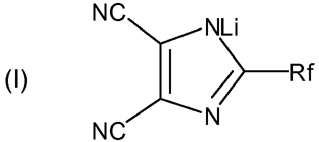 dans laquelle Rf représente un groupement alkyle fluoré ayant de 1 à 5 atome(s) de carbone, ou un atome de fluor et (ii) au moins un cation choisi dans le groupe A constitué par le sodium, le potassium, le calcium, le fer, le magnésium, le manganèse, le strontium, le vanadium, l'ammonium, l'argent, l'aluminium, l'arsenic, le barium, le silicium, le cadmium, le cobalt, le chrome, le cuivre, le nickel, le plomb, l'antimoine, le sélénium, l'étain, et le titane et (iii) au moins un anion choisi dans le groupe B constitué par le fluorure, le chlorure, le nitrate, le sulfate, le phosphate, l'acétate, le formiate, le trifluoroacétate, le pentafluoroacétate et l'anion de formule (II) 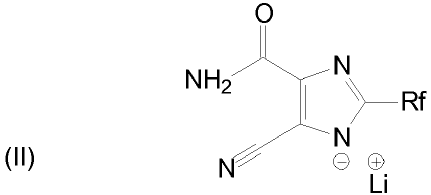 avec la quantité totale des cation(s) et anion(s) étant supérieure à 0% et d'au plus 1 % en poids de la composition.

2. Composition selon la revendication 1 comprenant au moins une des caractéristiques suivantes :
- la quantité de sodium est comprise entre 0 et 500 ppm et de préférence compris entre 0 et 100 ppm;
- la quantité de potassium est comprise entre 0 et 1000 ppm et de préférence comprise entre 0 et 500 ppm et avantageusement comprise entre 0 et 100 ppm;
- la quantité de calcium est comprise entre 0 et 70 ppm;
- la quantité de fer est comprise entre 0 et 10 ppm;
- la quantité de magnésium est comprise entre 0 et 10 ppm;
- la quantité de manganèse est comprise entre 0 et 5 ppm;
- la quantité de strontium est comprise entre 0 et 5 ppm;
- la quantité de vanadium est comprise entre 0 et 10 ppm;
- la quantité totale des cations suivants : Ag, Al, As, Ba, Si, Cd, Co, Cr, Cu, Ni, Pb, Sb, Se, Sn, Sr, Ti, Zn est comprise entre 0 et 200 ppm ;
- la quantité d'ammonium (NH₄⁺) est comprise entre 0 et 10 ppm;
- la quantité de fluorure est comprise entre 0 et 100 ppm et de préférence comprise entre 0 et 10 ppm;
- la quantité d'acétate est comprise entre 0 et 30 ppm et de préférence comprise entre 0 et 5 ppm;
- la quantité de formiate est comprise entre 0 et 200 ppm et de préférence comprise entre 0 et 10 ppm;
- la quantité de chlorure est comprise entre 0 et 500 ppm et de préférence comprise entre 0 et 100 ppm et avantageusement comprise entre 0 et 50 ppm;
- la quantité de nitrate est comprise entre 0 et 150 ppm et de préférence entre 0 et 100 ppm et avantageusement entre 0 et 50 ppm;
- la quantité de sulfate est comprise entre 0 et 500 ppm et de préférence comprise entre 0 et 150 ppm, et avantageusement comprise entre 0 et 25 ppm;
- la quantité de phosphate est comprise entre 0 et 100 ppm et de préférence entre 0 et 10 ppm;
- la quantité de trifluoroacétate est comprise entre 0 et 100 ppm ;
- la quantité de pentafluoroacétate est comprise entre 0 et 200 ppm et de préférence entre 0 et 100 ppm ;
- la quantité de l'anion de (II) est comprise entre 0 et 600 ppm et de préférence entre 0 et 400 ppm;
- Rf représente F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F_{7,} C₄H₄F₅, C₅F₁₁, C₃F₆OCF₃, C₂F₄OCF₃, C₂H₂F₂OCF₃ ou CF₂OCF₃, de préférence CF₃, C₂F₅, C₂F₄OCF₃, C₂H₂F₂OCF₃ ou CF₂OCF₃.

3. Composition selon la revendication 1 ou 2 **caractérisée en ce que** la valeur absolue de la déviation *a*, par rapport à l'horizontale, de la ligne de base du spectre Raman, prise entre 4500 cm⁻¹ et 3000 cm⁻¹, assimilée à une droite ayant pour équation *y* = *ax* + *b* est ≤ 25, de préférence ≤15 et avantageusement ≤ 5, ; y étant l'intensité, x étant la longueur d'onde, et b étant un décalage en intensité y à la longueur d'onde 4500 cm-1 dû à l'effet de la fluorescence causée par les traces d'impuretés.

4. Composition selon l'une quelconque des revendications précédentes **caractérisée en ce que** le sodium et/ou le potassium (est) sont présent(s) en quantité supérieure à 0 ppm et inférieure ou égale à 100 ppm.

5. Composition selon l'une quelconque des revendications précédentes **caractérisée en ce que** le trifluoroacétate et /ou le pentafluoroacétate (est) sont présent(s) en quantité supérieure à 0 ppm et inférieure ou égale à 100 ppm.

6. Composition selon l'une quelconque des revendications précédentes **caractérisée en ce que** le sel de formule (I) représente au moins 99 %, de préférence au moins 99,9 % et avantageusement au moins 99,95 % en poids de la composition.

7. Composition selon l'une quelconque des revendications précédentes **caractérisée en ce qu'**elle présente une coloration Hazen < 10 pour une concentration d'une mole pour le sel de formule (I) par litre d'un solvant, non absorbant dans le visible.

8. Composition selon l'une quelconque des revendications précédentes **caractérisée en ce que** le composé de formule (I) est le 2-trifluorométhyl-4,5-dicarbonitrile-imidazolate de lithium.

9. Procédé de préparation de la composition selon l'une quelconque des revendications précédentes **caractérisé en ce qu'**il comprend au moins une étape de traitement d'une solution de sel renfermant le composé de formule (I), préalablement préparée à partir du diaminomaléonitrile, sur du charbon actif suivie d'au moins une étape de recristillation de la solution traitée.

10. Procédé selon la revendication 9 **caractérisé en ce qu'**il comprend (i) au moins une étape de traitement d'une solution résultant de la réaction d'un composé_imidazole de formule (III), 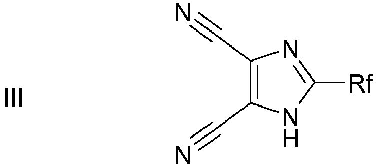 en présence d'un solvant (S), avec une base lithiée, de préférence choisie parmi l'hydrure de lithium, le carbonate de lithium, l'hydroxyde de lithium et les combinaisons de ceux-ci, sur du charbon actif ; (ii) au moins une étape de séparation du charbon actif pour donner une solution de composition de sel de formule (I) dans le solvant (S) et (iii) au moins une étape de concentration de la solution obtenue à l'étape (ii) suivie d'au moins une étape de refroidissement jusqu'à la formation de cristaux et (iv) au moins une étape d'essorage et/ou de filtration suivie d'au moins une étape de séchage.

11. Procédé selon la revendication 10 **caractérisé en ce que** la solution récupérée à l'étape (ii) est évaporée à sec, puis le solide obtenu est mis en solution dans un solvant (S), différent de celui utilisé pour l'étape (i), avant l'étape de cristallisation (iii) décrite ci-dessus.

12. Procédé selon la revendication 10 ou 11 **caractérisé en ce que** le solvant (S) est choisi parmi l'eau ou un solvant organique, de préférence l'acétonitrile, les alcools avantageusement le méthanol, l'éthanol, l'isopropanol, le n-butanol, l'alcool benzylique, l'acétate d'éthyle, les carbonates d'alkyle, tels que l'éthylène ou le diméthyle carbonates, et le tetrahydrofurane.

13. Procédé selon l'une quelconque des revendications 9 à 12 **caractérisé en ce que** le charbon actif est choisi parmi les grades de charbons commerciaux dits physiques, de préférence issus de matière première végétale dite « tendre ».

14. Procédé selon l'une quelconque des revendications 9 à 13 **caractérisé en ce que** le charbon actif est en poudre ou en grain, ou sous forme de media filtrants composites.

15. Procédé selon l'une quelconque des revendications 9 à 14 **caractérisé en ce que** la porosité du charbon actif, mesurée par N₂-BET, est comprise entre 900 et 1800 m²/g, de préférence comprise entre 900 et 1300 m²/g et avantageusement comprise entre 900 et 1100 m²/g.

16. Utilisation de la composition selon l'une quelconque des revendications 1 à 8 comme électrolyte, de préférence électrolyte de batterie.

## Patentansprüche

1. Zusammensetzung, umfassend (i) ein Lithiumsalz der Imidazol-Verbindung mit der Formel (I): 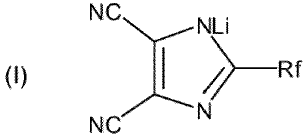 wobei Rf eine fluorierte Alkylgruppe mit 1 bis 5 Kohlenstoffatom(en), oder ein Fluoratom darstellt, und (ii) mindestens ein Kation, ausgewählt aus der Gruppe A, bestehend aus Natrium, Kalium, Calcium, Eisen, Magnesium, Mangan, Strontium, Vanadium, Ammonium, Silber, Aluminium, Arsen, Barium, Silicium, Cadmium, Kobalt, Chrom, Kupfer, Nickel, Blei, Antimon, Selen, Zinn und Titan, und (iii) mindestens ein Anion, ausgewählt aus der Gruppe B, bestehend aus Fluorid, Chlorid, Nitrat, Sulfat, Phosphat, Acetat, Formiat, Trifluoracetat, Pentafluoracetat und dem Anion der Formel (II): 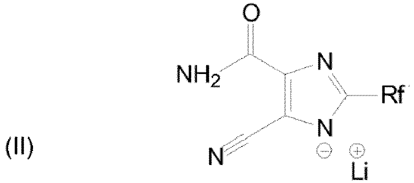 wobei die Gesamtmenge von Kation(en) und Anion(en) größer ist als 0 % und höchstens 1 Gew.-% der Zusammensetzung beträgt.

2. Zusammensetzung nach Anspruch 1, umfassend mindestens eine der folgenden Charakteristiken:
- die Menge an Natrium beträgt zwischen 0 und 500 ppm und vorzugsweise zwischen 0 und 100 ppm;
- die Menge an Kalium beträgt zwischen 0 und 1000 ppm und vorzugsweise zwischen 0 und 500 ppm und vorteilhaft zwischen 0 und 100 ppm;
- die Menge an Calcium beträgt zwischen 0 und 70 ppm;
- die Menge an Eisen beträgt zwischen 0 und 10 ppm;
- die Menge an Magnesium beträgt zwischen 0 und 10 ppm;
- die Menge an Mangan beträgt zwischen 0 und 5 ppm;
- die Menge an Strontium beträgt zwischen 0 und 5 ppm;
- die Menge an Vanadium beträgt zwischen 0 und 10 ppm;
- die Gesamtmenge der folgenden Kationen: Ag, Al, As, Ba, Si, Cd, Co, Cr, Cu, Ni, Pb, Sb, Se, Sn, Sr, Ti, Zn beträgt zwischen 0 und 200 ppm;
- die Menge an Ammonium (NH₄⁺) beträgt zwischen 0 und 10 ppm;
- die Menge an Fluorid beträgt zwischen 0 und 100 ppm und vorzugsweise zwischen 0 und 10 ppm;
- die Menge an Acetat beträgt zwischen 0 und 30 ppm und vorzugsweise zwischen 0 und 5 ppm;
- die Menge an Formiat beträgt zwischen 0 und 200 ppm und vorzugsweise zwischen 0 und 10 ppm;
- die Menge an Chlorid beträgt zwischen 0 und 500 ppm und vorzugsweise zwischen 0 und 100 ppm und vorteilhaft zwischen 0 und 50 ppm;
- die Menge an Nitrat beträgt zwischen 0 und 150 ppm und vorzugsweise zwischen 0 und 100 ppm und vorteilhaft zwischen 0 und 50 ppm;
- die Menge an Sulfat beträgt zwischen 0 und 500 ppm und vorzugsweise zwischen 0 und 150 ppm und vorteilhaft zwischen 0 und 25 ppm;
- die Menge an Phosphat beträgt zwischen 0 und 100 ppm und vorzugsweise zwischen 0 und 10 ppm;
- die Menge an Trifluoracetat beträgt zwischen 0 und 100 ppm;
- die Menge an Pentafluoracetat beträgt zwischen 0 und 200 ppm und vorzugsweise zwischen 0 und 100 ppm;
- die Menge an Anion (II) beträgt zwischen 0 und 600 ppm und vorzugsweise zwischen 0 und 400 ppm;
- Rf bedeutet F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F_{7,} C₄H₄F₅, C₅F₁₁, C₃F₆OCF₃, C₂F₄OCF₃, C₂H₂F₂OCF₃ oder CF₂OCF₃, vorzugsweise CF₃, C₂F₅, C₂F₄OCF₃, C₂H₂F₂OCF₃ oder CF₂OCF₃.

3. Zusammensetzung nach Anspruch 1 oder 2, **dadurch gekennzeichnet, dass**, in Bezug auf die Horizontale, der Absolutwert der Abweichung a der Basislinie des Raman-Spektrums, bestimmt zwischen 4500 cm⁻¹ und 3000 cm⁻¹, assimiliert an eine Gerade mit der Gleichung y = ax + b, ≤ 25 ist, vorzugsweise ≤ 15 und vorteilhaft ≤ 5; wobei y die Intensität ist, x die Wellenlänge ist und b eine Verschiebung der Intensität y ist bei der Wellenlänge von 4500 cm⁻¹ aufgrund des Effekts der Fluoreszenz, die von Verunreinigungsspuren verursacht wird.

4. Zusammensetzung nach einem der vorhergehenden Ansprüche, **dadurch gekennzeichnet, dass** Natrium und/oder Kalium in einer Menge von größer als 0 ppm und kleiner oder gleich 100 ppm vorhanden ist (sind).

5. Zusammensetzung nach einem der vorhergehenden Ansprüche, **dadurch gekennzeichnet, dass** Trifluoracetat und/oder Pentafluoracetat in einer Menge von größer als 0 ppm und kleiner oder gleich 100 ppm vorhanden ist (sind).

6. Zusammensetzung nach einem der vorhergehenden Ansprüche, **dadurch gekennzeichnet, dass** das Salz mit der Formel (I) mindestens 99 Gew.-%, vorzugsweise mindestens 99,9 Gew.-% und vorteilhaft mindestens 99,95 Gew.-%, der Zusammensetzung bildet.

7. Zusammensetzung nach einem der vorhergehenden Ansprüche, **dadurch gekennzeichnet, dass** diese eine Hazen-Färbung < 10 für eine Konzentration von einem Mol für das Salz mit der Formel (I) pro Liter eines Lösungsmittels, nicht-absorbierend im sichtbaren Bereich, aufweist.

8. Zusammensetzung nach einem der vorhergehenden Ansprüche, **dadurch gekennzeichnet, dass** die Verbindung mit der Formel (I) 2-Lithiumtrifluormethyl-4,5-dicarbonitrilimidazol ist.

9. Verfahren zur Herstellung der Zusammensetzung nach einem der vorhergehenden Ansprüche, **dadurch gekennzeichnet, dass** dieses mindestens einen Schritt der Behandlung einer Lösung eines Salzes, umfassend die Verbindung mit der Formel (I), zuvor hergestellt aus Diaminomaleonitril, auf Aktivkohle umfasst, gefolgt von mindestens einem Schritt der Umkristallisation der behandelten Lösung.

10. Verfahren nach Anspruch 9, **dadurch gekennzeichnet, dass** dieses umfasst: (i) mindestens einen Schritt der Behandlung einer Lösung, die erhalten wird aus dem Umsetzen einer Imidazol-Verbindung mit der Formel (III): 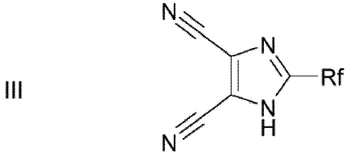 in Anwesenheit eines Lösungsmittels (S) mit einer Lithiumbase, vorzugsweise ausgewählt aus Lithiumhydrid, Lithiumcarbonat, Lithiumhydroxid und Kombinationen derselben, auf Aktivkohle; (ii) mindestens einen Schritt des Abtrennens der Aktivkohle, um eine Lösung der Zusammensetzung eines Salzes mit der Formel (I) im Lösungsmittel (S) zu ergeben; und (iii) mindestens einen Schritt der Konzentration der in Schritt (ii) erhaltenen Lösung, gefolgt von mindestens einem Schritt des Abkühlens bis zur Bildung von Kristallen; und (iv) mindestens einen Schritt des Entwässerns und/oder Filtrierens, gefolgt von mindestens einem Schritt der Trocknung.

11. Verfahren nach Anspruch 10, **dadurch gekennzeichnet, dass** die in Schritt (ii) gewonnene Lösung zur Trockene eingedampft wird, dann der erhaltene Feststoff in einem Lösungsmittel (S) gelöst wird, das von dem für Schritt (i) verwendeten verschieden ist, vor dem oben beschriebenen Kristallisationsschritt (iii).

12. Verfahren nach Anspruch 10 oder 11, **dadurch gekennzeichnet, dass** das Lösungsmittel (S) ausgewählt wird aus Wasser oder einem organischen Lösungsmittel, vorzugsweise Acetonitril, Alkoholen, vorteilhaft Methanol, Ethanol, Isopropanol, n-Butanol, Benzylalkohol, Ethylacetat, Alkylcarbonaten, wie Ethylen- oder Dimethylcarbonaten, und Tetrahydrofuran.

13. Verfahren nach einem der Ansprüche 9 bis 12, **dadurch gekennzeichnet, dass** die Aktivkohle aus Graden von kommerzieller Kohle ausgewählt wird, die als physikalisch bezeichnet werden, vorzugsweise stammend von pflanzlichem Ausgangsmaterial, das als "weich" bezeichnet wird.

14. Verfahren nach einem der Ansprüche 9 bis 13, **dadurch gekennzeichnet, dass** die Aktivkohle in Pulver- oder Kornform oder in der Form von Verbundfiltermedien ist.

15. Verfahren nach einem der Ansprüche 9 bis 14, **dadurch gekennzeichnet, dass** die Porosität der Aktivkohle, gemessen durch N₂-BET, zwischen 900 und 1800 m²/g, vorzugsweise zwischen 900 und 1300 m²/g und vorteilhaft zwischen 900 und 1100 m²g/g, beträgt.

16. Verwendung der Zusammensetzung nach einem der Ansprüche 1 bis 8 als Elektrolyt, vorzugsweise als Elektrolyt einer Batterie.

## Claims

1. Composition comprising (i) a lithium salt of an imidazole compound of formula (I): 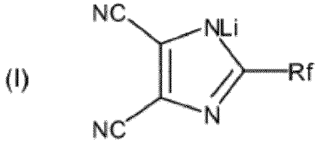 wherein Rf represents a fluorinated alkyl group having from 1 to 5 carbon atom(s), or a fluorine atom, and (ii) at least one cation chosen from the group A made up of sodium, potassium, calcium, iron, magnesium, manganese, strontium, vanadium, ammonium, silver, aluminum, arsenic, barium, silicon, cadmium, cobalt, chromium, copper, nickel, lead, antimony, selenium, tin and titanium, and (iii) at least one anion chosen from the group B made up of fluoride, chloride, nitrate, sulfate, phosphate, acetate, formate, trifluoroacetate, pentafluoroacetate and the anion of formula (II) 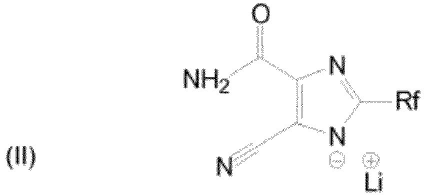 with the total amount of the cation(s) and anion (s) being more than 0% and at most 1% by weight of the composition.

2. Composition according to Claim 1, comprising at least one of the following characteristics:
- the amount of sodium is between 0 and 500 ppm and preferably between 0 and 100 ppm;
- the amount of potassium is between 0 and 1000 ppm and preferably between 0 and 500 ppm and advantageously between 0 and 100 ppm;
- the amount of calcium is between 0 and 70 ppm;
- the amount of iron is between 0 and 10 ppm;
- the amount of magnesium is between 0 and 10 ppm;
- the amount of manganese is between 0 and 5 ppm;
- the amount of strontium is between 0 and 5 ppm;
- the amount of vanadium is between 0 and 10 ppm;
- the total amount of the following cations: Ag, Al, As, Ba, Si, Cd, Co, Cr, Cu, Ni, Pb, Sb, Se, Sn, Sr, Ti, Zn is between 0 and 200 ppm;
- the amount of ammonium (NH₄⁺) is between 0 and 10 ppm;
- the amount of fluoride is between 0 and 100 ppm and preferably between 0 and 10 ppm;
- the amount of acetate is between 0 and 30 ppm and preferably between 0 and 5 ppm;
- the amount of formate is between 0 and 200 ppm and preferably between 0 and 10 ppm;
- the amount of chloride is between 0 and 500 ppm and preferably between 0 and 100 ppm and advantageously between 0 and 50 ppm;
- the amount of nitrate is between 0 and 150 ppm and preferably between 0 and 100 ppm and advantageously between 0 and 50 ppm;
- the amount of sulfate is between 0 and 500 ppm and preferably between 0 and 150 ppm and advantageously between 0 and 25 ppm;
- the amount of phosphate is between 0 and 100 ppm and preferably between 0 and 10 ppm;
- the amount of trifluoroacetate is between 0 and 100 ppm;
- the amount of pentafluoroacetate is between 0 and 200 ppm and preferably between 0 and 100 ppm;
- the amount of the anion of (II) is between 0 and 600 ppm and preferably between 0 and 400 ppm;
- Rf represents F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F_{7,} C₄H₄F₅, C₅F₁₁, C₃F₆OCF₃, C₂F₄OCF₃, C₂H₂F₂OCF₃ or CF₂OCF₃, preferably CF₃, C₂F₅, C₂F₄OCF₃, C₂H₂F₂OCF₃ or CF₂OCF₃.

3. Composition according to Claim 1 or 2, **characterized in that** the absolute value of the deviation *a*, relative to the horizontal, of the baseline of the Raman spectrum, taken between 4500 cm⁻¹ and 3000 cm⁻¹, comparable to a straight line having the equation *y* = *ax* + *b* is ≤ 25, preferably ≤ 15 and advantageously ≤ 5; y being the intensity, x being the wavelength, and b being a shift in intensity y at the wavelength 4500 cm⁻¹ due to the effect of the fluorescence caused by the traces of impurities.

4. Composition according to any one of the preceding claims, **characterized in that** the sodium and/or the potassium (is) are present in an amount greater than 0 ppm and less than or equal to 100 ppm.

5. Composition according to any one of the preceding claims, **characterized in that** the trifluoroacetate and/or the pentafluoroacetate (is) are present in an amount greater than 0 ppm and less than or equal to 100 ppm.

6. Composition according to any one of the preceding claims, **characterized in that** the salt of formula (I) represents at least 99%, preferably at least 99.9% and advantageously at least 99.95% by weight of the composition.

7. Composition according to any one of the preceding claims, **characterized in that** it has a Hazen color < 10 for a concentration of one mol for the salt of formula (I) per liter of a solvent, which is nonabsorbent in the visible range.

8. Composition according to any one of the preceding claims, **characterized in that** the compound of formula (I) is lithium 2-trifluoromethyl-4,5-dicarbonitrileimidazolate.

9. Process for preparing the composition according to any one of the preceding claims, **characterized in that** it comprises at least one step of treating a solution of salt containing the compound of formula (I), which has been previously prepared from diaminomaleonitrile, on activated carbon, followed by at least one step of recrystallization of the treated solution.

10. Process according to Claim 9, **characterized in that** it comprises (i) at least one step of treating a solution resulting from the reaction of an imidazole compound of formula (III), 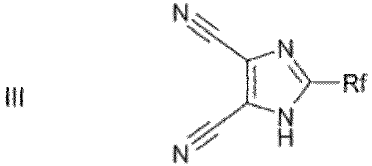 in the presence of a solvent (S), with a lithium base, preferably chosen from lithium hydride, lithium carbonate, lithium hydroxide and combinations thereof, on activated carbon; (ii) at least one step of separating the activated carbon so as to give a solution of composition of salt of formula (I) in the solvent (S); and (iii) at least one step of concentrating the solution obtained in step (ii), followed by at least one step of cooling until the formation of crystals and (iv) at least one filter-drying and/or filtering step, followed by at least one drying step.

11. Process according to Claim 10, **characterized in that** the solution recovered in step (ii) is evaporated to dryness, then the solid obtained is dissolved in a solvent (S), different than the one used for step (i), before the crystallization step (iii) described above.

12. Process according to Claim 10 or 11, **characterized in that** the solvent (S) is chosen from water or an organic solvent, preferably acetonitrile, alcohols, advantageously methanol, ethanol, isopropanol, n-butanol or benzyl alcohol, ethyl acetate, alkyl carbonates such as ethylene or dimethyl carbonates, and tetrahydrofuran.

13. Process according to any one of Claims 9 to 12, **characterized in that** the activated carbon is chosen from the commercially available grades of carbons termed physical carbons, preferably derived from "soft" vegetable raw material.

14. Process according to any one of Claims 9 to 13, **characterized in that** the activated carbon is powdered or in the form of grains, or in the form of composite filtering media.

15. Process according to any one of Claims 9 to 14, **characterized in that** the porosity of the activated carbon, measured by N₂-BET, is between 900 and 1800 m²/g, preferably between 900 and 1300 m²/g and advantageously between 900 and 1100 m²/g.

16. Use of the composition according to any one of Claims 1 to 8 as an electrolyte, preferably a battery electrolyte.
